# EUROPEAN PATENT SPECIFICATION

(11) **EP 3 868 218 B1**
(45) Date of publication and mention of the grant of the patent: **05.07.2023**
(21) Application number: 19873759.5
(22) Date of filing: 03.09.2019
(51) Int. Cl.: A23L 33/105, A61K 36/23, A61K 9/00, A61P 27/02, A61P 27/06

(54) **COMPOSITION FOR PREVENTING OR TREATING RETINAL DISEASE, CONTAINING CENTELLA ASIATICA EXTRACT**
ZUSAMMENSETZUNG ZUR VORBEUGUNG ODER BEHANDLUNG VON RETINAERKRANKUNGEN MIT EXTRAKT AUS CENTELLA ASIATICA
COMPOSITION PERMETTANT DE PRÉVENIR OU DE TRAITER UNE MALADIE RÉTINIENNE CONTENANT UN EXTRAIT DE CENTELLA ASIATICA

(30) Priority: 16.10.2018 KR 20180122947
(43) Date of publication of application: 25.08.2021
(73) Proprietor: Genencell Inc., Yongin-si, Gyeonggi-do 16950 (KR)
(72) Inventor: JEONG, Yong Joon, Suwon-si Gyeonggi-do 16519 (KR); KANG, Se Chan, Seongnam-si Gyeonggi-do 13608 (KR); PARK, Dae Won, Seoul 01033 (KR); SO, Ri Na, Seoul 08727 (KR)
(74) Representative: WSL Patentanwälte Partnerschaft mbB
(86) International application number: PCT/KR2019/011310
(87) International publication number: WO 2020/080673

(56) References cited:
- WO-A1-2017/130130
- KR-A- 20130 133 338
- KR-A- 20170 036 352
- KR-A- 20170 102 861
- US-A1- 2014 141 082
- HUANG WANJING ET AL: "Asiatic Acid Prevents Retinal Ganglion Cell Apoptosis in a Rat Model of Glaucoma", FRONTIERS IN NEUROSCIENCE, vol. 12, 20 July 2018 (2018-07-20), XP055917842, DOI: 10.3389/fnins.2018.00489
- YANG BOYU ET AL: "Madecassic Acid protects against hypoxia-induced oxidative stress in retinal microvascular endothelial cells via ROS-mediated endoplasmic reticulum stress", BIOMEDICINE & PHARMACOTHERAPY, ELSEVIER, FR, vol. 84, 8 October 2016 (2016-10-08), pages 845-852, XP029828724, ISSN: 0753-3322, DOI: 10.1016/J.BIOPHA.2016.10.015
- Yong Joon Jeong, Hyun Jin Baek, Dae Won Park, Se Min Jo, Hyelin Jeon, Se Chan Kang: "11-018 Protection effect of Centella asiatica extracts in the retinal cell", 2018 KoSFoST International Symposium and Annual Meeting, 27-29 June, 2018, Busan (Korea), 27 June 2018 (2018-06-27), page 519, XP009526399,
- GENENCELL CO., LTD.: "Identical Standard Eye/Liver Health Multi-Functional Individual Recognition Type Health Functional Food", InterBiz Bio-Partnering & Investment Forum 2018, 4 July 2018 (2018-07-04),

## Description

### [Technical Field]

The present invention relates to a novel use of a *Centella asiatica* extract for protecting retinal nerve cells from oxidative stress, improving the survival rate of retinal nerve cells or retinal pigment epithelial cells through increased sugar availability, inhibiting the apoptosis of retinal nerve cells, or preventing, ameliorating, or treating glaucoma or macular degeneration.

### [Background Art]

Glaucoma is a disease caused by damage to the optic nerve due to an increase in intraocular pressure or abnormal blood circulation of the optic nerve, is characterized by temporary or permanent visual field defects and decreased vision, and may lead to permanent blindness if not treated. The general cause of the onset thereof is increased intraocular pressure due to circulatory disorders of the aqueous humor, and at the age group of 40s or older, an aqueous humor outlet becomes narrower and the amount of aqueous humor produced increases, resulting in increased intraocular pressure, which causes pressure on and damage to the optic nerve. In addition, although intraocular pressure is normal, glaucoma develops in some cases, and especially in Korea and Japan, there are more patients with glaucoma with normal intraocular pressure (about 70-80% of glaucoma patients), and the cause is known as an optic nerve blood circulation disorder. Practically, it is known that there are many cases of peripheral blood circulation disorders caused by metabolic diseases in patients within the range of normal intraocular pressure. Ultimately, glaucoma leads to the death of optic nerve cells such as retinal node cells (Almasieh et al., 2012), and substances capable of preventing and treating the death of optic nerve cells can be used as foods and therapeutic agents for inhibiting the onset and progression of glaucoma.

The macula is the part of the eye's retina that receives light most clearly and accurately because optic cells are concentrated, and plays a very important role in vision, and the macular is degenerated by various causes, causing vision disorders, which is referred to as macular degeneration. Macular degeneration is a disease caused by gradual degeneration of retinal epithelial cells, the retina, and the capillary layer of the choroid, and in early stages, macular degeneration is characterized by observation of abnormal retinal epithelial cells in which extracellular deposits called drusen are formed between the Bruch's membrane and retinal epithelial cells. In addition, in later stages, macular degeneration is broadly divided into exudative (wet) macular degeneration and atrophic (dry) macular degeneration, and in both types of macular degeneration, optic cells in the macula are gradually destroyed, and thus the function of the macula deteriorates over time and vision at the central portion decreases. The main etiology of macular degeneration is known to be oxidative stress and inflammation, and particularly, when A2E molecules, which are by-products after intracellular metabolism, are not excreted in retinal pigment epithelial cells and gradually accumulate, they undergo an oxidative stress process and eventually leads to cell death (Sparrow and Cai, 2001). Thus, it is known that, since blocking the accumulation of the causative agent A2E molecule itself can prevent oxidation in retinal cells, the risk itself of macular degeneration can be reduced.

Glaucoma and macular degeneration are one of the three major causes of blindness, along with diabetic retinopathy, and the death of optic nerve cells or retinal nerve cells can be the main cause, and it is known that these two diseases are rapidly increasing in patients in their 30s to 50s as well as the elderly due to the recent increase in the prevalence of metabolic diseases and the like.

Recently, medicines and health functional foods for maintaining eye health such as the prevention of oxidative damage to the eyes have been actively developed, and particularly, energy improvement agents using wild blueberry-derived anthocyanoside as a main ingredient have been developed, and herbal medicines such as cassia seeds and *Lycium* have long been used for vision maintenance and vision improvement. However, there are no studies on a method of preventing or treating glaucoma and macular degeneration of the retina, which are related to eye health, using *Centella asiatica.*

### [Cited References]

### [Patent Documents]

(Patent Document 1) Korean Patent Registration No. 10-1700903
(Patent Document 2) Korean Patent Publication No. 10-2018-0080584

HUANG WANJING ET AL. ("FRONTIERS IN NEUROSCIENCE, vol. 12, 20 July 2018) disclose that asiatic acid, a triterpene derived from *Centella asiatica,* prevents retinal ganglion cell apoptosis in a rat model of glaucoma.

YANG BOYU ET AL. (BIOMEDICINE & PHARMACOTHERAPY, ELSEVIER, FR, vol. 84, 8 October 2016, pages 845-852) disclose that madecassic acid, a triterpene derived from *Centella asiatica,* protects against hypoxia-induced oxidative stress in retinal microvascular endothelial cells.

Yong Joon Jeong, ET AL. (2018 KoSFoST International Symposium and Annual Meeting, 27-29 June, 2018, Busan (Korea)) disclose a protection effect of *Centella asiatica* extracts in the retinal cell.

### [Disclosure]

### [Technical Problem]

Therefore, the inventors of the present invention have recognized the importance of prevention and treatment of glaucoma, macular degeneration, and the like related to eye health, and used various natural substances to select natural resources exhibiting proliferative efficacy on retinal nerve cells and retinal pigment epithelial cells and capable of protecting oxidative damage to retinal cells. As a result, a *Centella asiatica* extract was found to exhibit excellent proliferative efficacy on optic nerve cells and retinal cells, and particularly, to inhibit oxidative damage caused by A2E in retinal cells. In addition, the *Centella* asiatica extract has greatly increased sugar availability in retinal cells, which indicates excellent effects of recovery and protection from cell damage, and thus the *Centella asiatica* extract of the present invention has a very high possibility of commercialization as a composition for preventing, ameliorating, or treating glaucoma and macular degeneration.

Therefore, the present invention has been made in view of the above problems, and it is an object of the present invention to provide a pharmaceutical composition for preventing, ameliorating, or treating glaucoma or macular degeneration including a *Centella asiatica* extract.

The objects of the present invention are not limited to the aforementioned objects. Other objects of the present invention will become more apparent from the following description, and will be realized by the means described in the claims and combinations thereof.

### [Technical Solution]

In accordance with an aspect of the present invention, the above and other objects can be accomplished by the provision of a food composition for protecting retinal nerve cells from oxidative stress, including a *Centella asiatica* extract as an active ingredient.

In one embodiment of the present invention, the *Centella asiatica* extract improves the survival rate of retinal nerve cells or retinal pigment epithelial cells through increased sugar availability.

In one embodiment of the present invention, the *Centella asiatica* extract inhibits the apoptosis of retinal nerve cells.

In one embodiment of the present invention, the *Centella asiatica* extract prevents the death of retinal nerve cells from oxidative stress caused by A2E.

In one embodiment of the present invention, the food composition prevents or ameliorates a retinal nerve cell damage syndrome caused by oxidative stress.

In accordance with another aspect of the present invention, there is provided a pharmaceutical composition for preventing, ameliorating, or treating a retinal nerve cell damage syndrome caused by oxidative stress, including a *Centella asiatica* extract as an active ingredient.

In one embodiment of the present invention, the *Centella asiatica* extract improves the survival rate of retinal nerve cells or retinal pigment epithelial cells through increased sugar availability.

In another embodiment of the present invention, the *Centella asiatica* extract inhibits the apoptosis of retinal nerve cells.

In another embodiment of the present invention, the *Ccentella asiatica* extract prevents the death of retinal nerve cells from oxidative stress caused by A2E.

In another embodiment of the present invention, the disease is macular degeneration or glaucoma.

In one or more embodiments of the present invention, the *Centella asiatica* extract is an extract obtained through extraction with water, a C₁₋₆ lower alcohol, or a mixed solvent thereof.

According to the present invention, the *Centella asiatica* extract is a 40%-60% aqueous ethanol extract.

In one or more embodiments of the present invention, the *Centella asiatica* extract is administered at a dose of 0.01 mg/kg/day to 10 g/kg/day.

In another embodiment of the present invention, the composition is a composition for topical administration.

In another embodiment of the present invention, the composition is an ophthalmic formulation.

In one or more embodiments of the present invention, the *Centella asiatica* extract is included in the composition at a concentration of 5 µg/ml to 500 µg/ml with respect to the total weight of the composition.

### [Advantageous Effects]

The *Centella asiatica* extract of the present invention effectively promotes the proliferation of retinal nerve cells and retinal pigment epithelial cells by increasing glucose uptake, and protects retinal cells from oxidative damage caused by A2E. Thus, the *Centella asiatica* extract can ameliorate, prevent, or treat eye diseases caused by damage to retinal nerve cells and retinal pigment epithelial cells, i.e., glaucoma and macular degeneration.

### [Description of Drawings]

The above and other objects, features and other advantages of the present invention will be more clearly understood from the following detailed description taken in conjunction with the accompanying drawings, in which:
FIG. 1 illustrates the cell growth efficacy of a *Centella asiatica* extract of the present invention in retinal nerve cells according to extraction solvent.
FIG. 2 illustrates cell viability in retinal nerve cells and retinal pigment epithelial cells by treatment with a composition of the present invention.
FIG. 3 illustrates glucose uptake efficacy in retinal pigment epithelial cells by treatment with a composition of the present invention.

### [Best Mode]

Unless otherwise specified, all numbers, figures, and/or expressions that represent ingredients, reaction conditions, and contents of ingredients used in the specification are approximations that reflect various uncertainties of measurement occurring inherently in obtaining these figures, among other things. For this reason, it should be understood that, in all cases, the term "about" should be considered to modify all numbers, figures, and/or expressions. In addition, when numeric ranges are disclosed in the description, these ranges are continuous and include all numbers from the minimum to the maximum including the maximum within the range, unless otherwise defined. Furthermore, when the range refers to an integer, it includes all integers from the minimum to the maximum including the maximum within the range, unless otherwise defined.

It should be understood that, in the specification, when the range refers to a parameter, the parameter encompasses all figures including end points disclosed within the range. For example, the range of "5 to 10" includes figures of 5, 6, 7, 8, 9, and 10, as well as arbitrary sub-ranges such as ranges of 6 to 10, 7 to 10, 6 to 9, and 7 to 9, and any figures, such as 5.5, 6.5, 7.5, 5.5 to 8.5, and 6.5 to 9, between appropriate integers that fall within the range. In addition, for example, the range of "10% to 30%" encompasses all integers that include figures such as 10%, 11%, 12%, and 13%, as well as 30%, and any sub-ranges of 10% to 15%, 12% to 18%, or 20% to 30%, as well as any figures, such as 10.5%, 15.5%, and 25.5%, between appropriate integers that fall within the range.

Hereinafter, the present invention will be described in detail.

The present invention relates to a composition for preventing, ameliorating, or treating glaucoma and macular degeneration, including a *Centella asiatica* extract. Specifically, the composition of the present invention induces the proliferation of retinal nerve cells and retinal pigment epithelial cells by increasing glucose uptake and protects cells from oxidative stress caused by A2E, and may be effectively used as a composition for preventing, ameliorating, or treating glaucoma and macular degeneration for eye health.

*Centella asiatica* is a perennial creeping herb belonging to the family *Umbelliferae,* and grows naturally in hot and humid areas, and in oriental medicine has been used as a herbal medicine for skin diseases, antipyretic action, hemoptysis, diuresis, tonicity, agenesis, leukorrhea, arthritis, and the like (Youngno LEE, 2006) . As known main ingredients of *Centella asiatica,* triterpenic acid sugar esters such as asiaticoside, madecassoside, brahmoside, and brahminoside are reported, and it is known that, when they are hydrolyzed, asiatic acid, madecassic acid, thankunic acid, isothankunic acid, and the like are produced. Asiaticoside and madecassoside, which are major ingredients of *Centella asiatica* and are pentacyclic triterpene glycosides belonging to the α-amyrin-ursolic acid group, have long been used for the treatment of skin wounds, chronic ulcers, or the like, and are reported to have leprosy treatment efficacy, antimicrobial efficacy, and therapeutic effects on wounds, gastric ulcers, various skin diseases, mental diseases, tuberculosis, venous diseases, dementia, and the like. Due to these effects, *Centella asiatica* is used in various applications such as a food product, a skin therapeutic agent, a wound therapeutic agent, a memory enhancement agent, and a tonic, and even in Korea, is used in fields such as pharmaceuticals, cosmetics, and functional foods, but no literature published to date has revealed that a *Centella asiatica* extract and main ingredients thereof are effective in preventing, ameliorating, or treating glaucoma and macular degeneration.

The present invention provides a pharmaceutical composition for preventing or treating glaucoma and macular degeneration, including a *Centella asiatica* extract. The composition may be a liquid extract or a lyophilized powder form of the liquid extract.

of the present invention, the *Centella asiatica* extract improves the survival rate of retinal nerve cells or retinal pigment epithelial cells through increased sugar availability.

In one embodiment of the present invention, the *Centella asiatica* extract inhibits the apoptosis of retinal nerve cells.

In one embodiment of the present invention, the *Centella asiatica* extract prevents the death of retinal nerve cells from oxidative stress caused by A2E.

In one embodiment of the present invention, the food composition prevents or ameliorates a retinal nerve cell damage syndrome caused by oxidative stress.

Another embodiment of the present invention provides a pharmaceutical composition for preventing, ameliorating, or treating a retinal nerve cell damage syndrome caused by oxidative stress, including a *Centella asiatica* extract as an active ingredient.

In one embodiment of the present invention, the *Centella asiatica* extract improves the survival rate of retinal nerve cells or retinal pigment epithelial cells through increased sugar availability.

In another embodiment of the present invention, the *Centella asiatica* extract inhibits the apoptosis of retinal nerve cells.

In another embodiment of the present invention, the *Centella asiatica* extract prevents the death of retinal nerve cells due to oxidative stress caused by A2E.

In another embodiment of the present invention, the disease is macular degeneration or glaucoma.

When the pharmaceutical composition is used clinically, the pharmaceutical composition may be mixed with general carriers in the pharmaceutical field and formulated as general preparations in the pharmaceutical field, for example, various preparations such as preparations for oral administration such as tablets, capsules, powders, granules, pills, liquids, and suspensions, injectable preparations in the form of a ready-to-use-type injectable dry powder that can be prepared and used as an injectable solution or suspension or distilled water injectable at the time of injection, or ointments. Pharmaceutical preparations prepared using general carriers may be administered orally, or may be applied parenterally, for example, intravenously, subcutaneously, intraperitoneally, or topically. Thus, the pharmaceutical composition of the present invention may further include suitable carriers, excipients, and diluents commonly used in the preparation of drugs.

A carrier, an excipient, and a diluent that may be included in the pharmaceutical composition of the present invention may include one or more selected from lactose, dextrose, sucrose, sorbitol, mannitol, xylitol, erythritol, maltitol, starch, acacia gum, alginates, gelatin, calcium phosphate, calcium silicate, cellulose, methyl cellulose, hydroxymethyl cellulose, microcrystalline cellulose, silicified microcrystalline cellulose, povidone, crospovidone, croscarmellose sodium, polyvinylpyrrolidone, water, methylhydroxybenzoate, propylhydroxybenzoate, Neusilin, colloidal silicon dioxide, lactose, talc, magnesium stearate, colloidal stearyl magnesium, and mineral oil.

The pharmaceutical composition is formulated using generally used diluents or excipients such as fillers, extenders, binders, wetting agents, disintegrating agents, and surfactants. Solid preparations for oral administration include tablets, pills, powders, granules, troches, lozenges, capsules, and such solid preparations are formulated by mixing the composition with at least one excipient, e.g., lactose, saccharose, sorbitol, mannitol, starch, amylopectin, cellulose, calcium carbonate, or gelatin. In addition to simple excipients, lubricants such as magnesium stearate and talc are also used. Examples of liquid preparations for oral administration include suspensions, liquids for internal use, emulsions, elixirs, syrups, and these liquid preparations may include, in addition to simple commonly used diluents such as water and liquid paraffin, various excipients, for example, a wetting agent, a sweetener, a flavoring agent, a preservative. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate. Examples of suppository bases include Witepsol, Macrogol, Tween 61, cacao butter, laurin, glycerogelatin. Parenteral administration may be generally performed via subcutaneous injection, intravenous injection, intramuscular injection, or intrathoracic injection.

A suitable dose of the pharmaceutical composition of the present invention varies depending on the age and body weight of the patient, the severity of the disease, the drug form, and the administration route and period, but may be appropriately selected by those of ordinary skill in the art. To obtain desired effects, the pharmaceutical composition of the present invention may be administered in an amount of 0.01 mg/kg/day to 10 g/kg/day, preferably 1 mg/kg/day to 1 g/kg/day. The pharmaceutical composition may be administered multiple times a day, preferably one to six times a day, at certain time intervals in accordance with the determination of a doctor or a pharmacist.

In one embodiment, the composition may be safely used for preventing, ameliorating, or treating glaucoma and macular degeneration as a natural product capable of being ingested and taken on a daily basis.

The *Centella asiatica* extract may be obtained by a conventional extraction method, and the extract may be an extract having a powder form through drying under reduced pressure and freeze drying.

In general, a *Centella asiatica* extract may be obtained through extraction with water, a C₁₋₆ lower alcohol, or a mixed solvent thereof.

In the composition of the present invention for preventing, ameliorating, or treating glaucoma and macular degeneration, which has the above-described composition, the extraction process may be repeated as needed, and an extract obtained by performing extraction 2 to 5 times may be used. In addition, a dry powder-type extract may be prepared by freeze-drying the extract, and may be used in a composition for preventing, ameliorating, or treating glaucoma and macular degeneration.

In general, it is desirable from the aspect of extraction efficiency that the content of alcohol in the extraction solvent be maintained at a concentration of 0 wt% to 95 wt%, preferably 30 wt% to 95 wt%, and more preferably 50 wt%. Any alcohol may be applied, as long as it is generally used in the art, preferably a C₁₋₅ alcohol. The alcohol may be one or more selected from methanol, ethanol, isopropanol, propanol, and butanol. According to the present invention, ethanol is used as the alcohol.

Particularly, according to the present invention, the *Centella asiatica* extract is a 40%-60% aqueous ethanol extract.

The extraction method is a method commonly known in the art, for example, a method using an extraction device, such as supercritical extraction, subcritical extraction, high-temperature extraction, high-pressure extraction, or ultrasonic extraction, a method using an adsorption resin including XAD and HP-20, or the like.

In addition, the extract is concentrated under reduced pressure using a vacuum rotary evaporator or the like to obtain an extract. In addition, the obtained extract may also be subjected to drying under reduced pressure, vacuum drying, boiling drying, spray drying, room-temperature drying, freeze drying, or the like as needed. In particular, the freeze-drying method is advantageous in that the loss of volatile organic substances in the extract can be reduced.

According to the present invention, the *Centella asiatica* extract is a 40% to 60% aqueous ethanol solution extract.

In one embodiment of the present invention, the composition promotes the proliferation of optic nerve cells and retinal cells or protects cells from oxidative stress, for eye health.
In one or more embodiments of the present invention, the *Centella asiatica* extract is administered at a dose of 0.01 mg/kg/day to 10 g/kg/day.

In another embodiment of the present invention, the composition is a composition for topical administration.

In another embodiment of the present invention, the composition is an ophthalmic formulation.

In one or more embodiments of the present invention, the *Centella asiatica* extract is included in the composition at a concentration of 5 µg/ml to 500 µg/ml with respect to the total weight of the composition.

The composition has an effect of effectively proliferating retinal nerve cells and retinal pigment epithelial cells and protecting the cells from oxidative stress. In addition, the composition may be safely used for preventing, ameliorating, or treating glaucoma and macular degeneration as a natural product capable of being ingested and taken on a daily basis.

The *Centella asiatica* extract may be obtained using a conventional extraction method, and the extract may be an extract in a powder form, obtained through drying under reduced pressure and freeze drying.

According to the present invention, the extract is a an aqueous alcohol solution extract.

In the composition of the present invention for preventing, ameliorating, or treating glaucoma and macular degeneration, which has the above-described composition, the extraction process may be repeated as needed, and an extract obtained by performing extraction 2 to 5 times may be used. In addition, a dry powder-type extract may be prepared by freeze-drying the extract, and may then be used in a composition for preventing, ameliorating, or treating glaucoma and macular degeneration.

It is desirable from the aspect of extraction efficiency that the content of alcohol in the extraction solvent be maintained at a concentration of 0 wt% to 95 wt%, preferably 30 wt% to 95 wt%, and more preferably 50 wt%. Any alcohol may be applied as long as it is one generally used in the art, preferably a C₁₋₅ alcohol. The alcohol may be one or more selected from methanol, ethanol, isopropanol, propanol, and butanol. More preferably, ethanol or the like may be used as the alcohol.

The extraction method is a method commonly known in the art, for example, a method using an extraction device, such as supercritical extraction, subcritical extraction, high-temperature extraction, high-pressure extraction, or ultrasonic extraction, a method using an adsorption resin including XAD and HP-20, or the like.

In addition, the extract is concentrated under reduced pressure using a vacuum rotary evaporator or the like to obtain an extract. In addition, the obtained extract may also be subjected to drying under reduced pressure, vacuum drying, boiling drying, spray drying, room-temperature drying, freeze drying, or the like as needed. In particular, the freeze-drying method is advantageous in that the loss of volatile organic substances from the extract can be reduced.

The *Centella asiatica* extract obtained through the above method effectively increases the proliferation of optic nerve cells and retinal cells by increasing the glucose uptake of cells, and effectively inhibits cell death from oxidative stress caused by A2E.

According to the present invention, the *Centella asiatica* extract is a 40% to 60% aqueous ethanol solution extract.

### [Mode for Invention]

Hereinafter, the present invention will be described in more detail with reference to specific examples. These examples are provided for illustrative purposes only to aid understanding of the present invention and are not intended to limit the scope of the present invention.

### [Example and Comparative Examples]

### Preparation Example: Preparation of Centella asiatica Extract

*Centella asiatica* was purchased from a farmhouse in Hapcheon, Gyeongnam and dried in the shade, the whole plant was pulverized, and then 1 kg of pulverized *Centella asiatica* was extracted with 50% alcohol for 5 hours at 80 °C, concentrated, and freeze-dried to thereby obtain 135 g (yield: 13.5%) of a *Centella asiatica* extract.

### Comparative Examples 1 to 4

As comparative examples, a hot-water extract of *Centella asiatica* (Comparative Example 1), a 30% alcohol extract of *Centella asiatica* (Comparative Example 2), a 70% alcohol extract of *Centella asiatica* (Comparative Example 3) and a 95% alcohol extract of *Centella asiatica* (Comparative Example 4) were used.

### [Experimental Examples]

### Experimental Example 1: Measurement of Effect on Proliferation of Retinal Nerve Cells and Retinal Pigment Epithelial Cells

In order to select the extract having the strongest efficacy from among the *Centella asiatica* extract obtained in Preparation Example and the extracts of Comparative Examples, the effect of proliferating retinal nerve cells was measured.

RGC-5 retinal nerve cells were incubated in a DMEM medium containing 10% serum and an antibiotic in an incubator supplied with 5 vol% of carbon dioxide and maintained at 37 °C. GH3 cells were dispensed into a 96-well plate at a concentration of 5 × 10³ cells/well and cultured for 24 hours. In order to measure the effect of each *Centella asiatica* extract on the proliferation of retinal nerve cells, the medium was replaced with a DMEM medium containing 10% charcoaled FBS, and then the cells were treated with 100 ug/mL of each of the *Centella asiatica* extracts prepared according to Preparation Example and Comparative Examples 1-4. After 48 hours, the growth rate of RGC-5 cells was measured at an absorbance of 550 nm using an MTT reagent, and the absorbance of each test solution was measured based on the control to represent a cell proliferation rate (%).

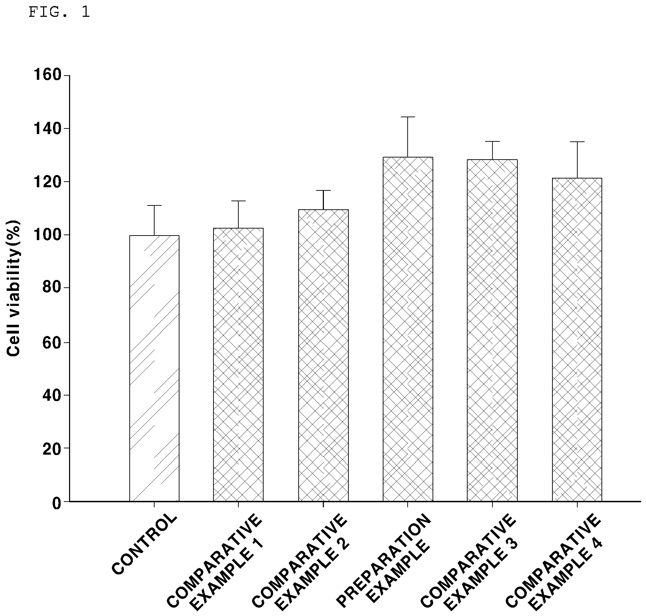

The proliferation rate of retinal nerve cells by each *Centella asiatica* extract is illustrated in FIG. 1.

As illustrated in FIG. 1, the 50% alcohol extract of *Centella asiatica* of Preparation Example exhibited the strongest effect on the proliferation of retinal nerve cells, and significant proliferative efficacy was also observed in Comparative Examples 3 and 4.

Meanwhile, in order to measure the effect of the 50% alcohol extract of *Centella asiatica,* which exhibited the strongest proliferative efficacy, on the protection of retinal nerve cells and retinal pigment epithelial cells, the effect on the proliferation of the corresponding retinal cells was measured.

RGC-5 retinal nerve cells were incubated in the same manner as described above, and ARPE-19 retinal pigment epithelial cells were incubated in a DMEM/F12 medium containing 10% serum and an antibiotic in an incubator supplied with 5 vol% of carbon dioxide and maintained at 37 °C. The RGC-5 cells and the ARPE-19 cells were dispensed into a 96-well plate at a concentration of 5 × 10³ cells/well and cultured for 24 hours. To measure the effect of the *Centella asiatica* extract on the proliferation of retinal nerve cells and retinal pigment epithelial cells, the medium was replaced with a DMEM medium containing 10% charcoaled FBS and a DMEM/F12 medium, and then the cells were treated with 50 µg/mL, 100 ug/mL, and 200 ug/mL of the 50% alcohol extract of *Centella asiatica.* After 48 hours, the growth rates of the respective cells were measured at an absorbance of 550 nm using an MTT reagent, and the absorbance of each test solution was measured and compared with the control to represent a cell proliferation rate (%).

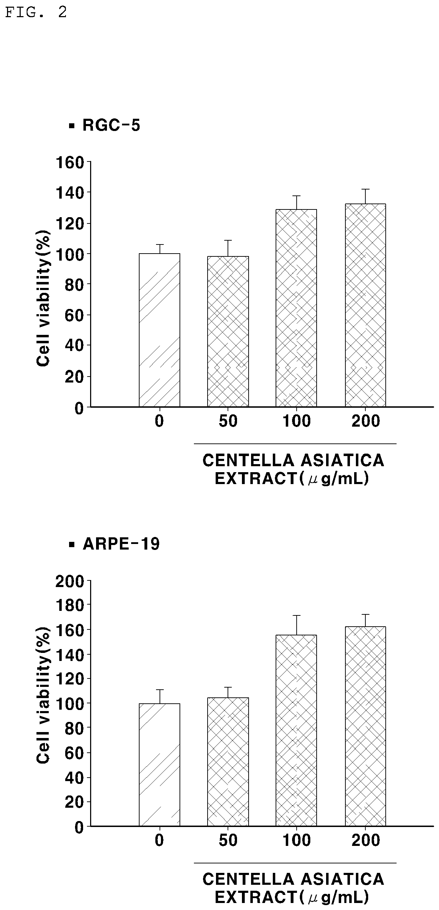

The proliferation rates of retinal nerve cells and retinal pigment epithelial cells due to the 50% alcohol extract of *Centella asiatica* are illustrated in FIG. 2.

As illustrated in FIG. 2, the 50% alcohol extract of *Centella asiatica* of Preparation Example exhibited the strongest proliferative efficacy at a concentration of 100 ug/mL or more.

### Experimental Example 2: Measurement of Glucose Uptake Efficacy in Retinal Pigment Epithelial Cells

A 2-NBDG uptake experiment was conducted to study the glucose uptake efficacy of the 50% alcohol extract of *Centella asiatica* of Preparation Example in retinal pigment epithelial cells.

As test cells, ARPE-19 cells, which are retinal pigment epithelial cells, were used, and the medium, medium composition, and experimental conditions were the same as in Experimental Example 1.

ARPE-19 cells were dispensed into a 96-well plate at a concentration of 1 × 10⁴ cells/well and cultured for 24 hours. To measure the glucose uptake efficacy, the cells were treated with 25 ug/mL, 50 ug/mL, and 100 ug/mL of the 50% alcohol extract of *Centella asiatica* and cultured for 24 hours, followed by treatment with a serum-free DMEM/F12 medium and 100 µM of 2-NBDG for 30 minutes. The cells were washed twice with cold phosphate-buffered saline (PBS), and then the glucose uptake amount was measured using a fluorescence photometer (excitation 485 nm; emission 525 nm) .

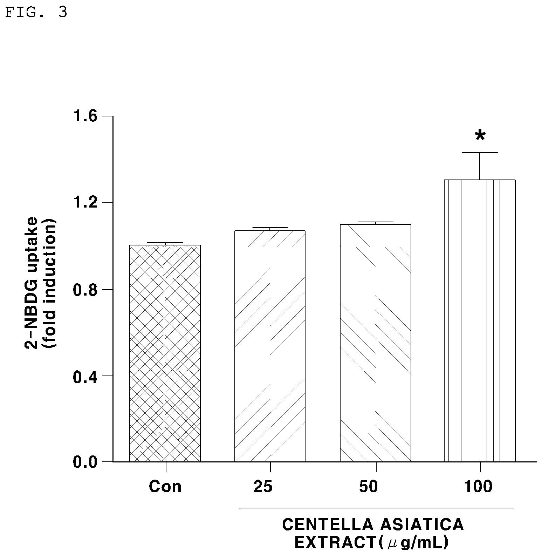

The glucose uptake efficacy of the *Centella asiatica* extract is shown in FIG. 3.

As illustrated in FIG. 3, very strong glucose uptake efficacy was shown at a concentration of 100 ug/mL of the *Centella asiatica* extract, which is considered to be evidence of the efficacy of the *Centella asiatica* extract on the proliferation of retinal cells.

### Experimental Example 3. Cell Protective Effect by A2E

To determine the effect of inhibiting macular degeneration, the cell death inhibitory effect of A2E was measured.

As test cells, ARPE-19 cells, which are retinal pigment epithelial cells, were used, and the medium, medium composition, and experimental conditions were the same as in Experimental Example 1.

ARPE-19 cells were dispensed into a 24-well plate at a concentration of 3 × 10⁴ cells/well and cultured for 24 hours, and then A2E was accumulated at a concentration of 3 µM for 3 days. In order to measure the effect of protecting cells from oxidative stress caused by A2E, the cells were treated with 25 µg/mL, 50 µg/mL, and 100 ug/mL of the 50% alcohol extract of *Centella asiatica,* and as a positive control, the cells were treated with 30 µM of lutein, which is known to have an inhibitory effect on macular degeneration, and cultured for 48 hours, and after 48 hours, the cells were irradiated with ultraviolet rays using a UV lamp for 5 minutes. The cell growth rate was measured at an absorbance of 570 nm using an MTT reagent, and the absorbance of each test solution was measured and compared with a control not treated with A2E.

The effect of the *Centella asiatica* extract on the protection of cells from oxidative stress caused by A2E is shown in Table 1.

**[Table 1]**

| | |
|---|---|
| | |

| Control (group not treated with A2E) | |
|---|---|
| Control | 61.4 ± 8.00 |
| Lutein (30 µM) | 71.5 ± 8.22 |
| 25 µg/mL of *Centella asiatica* | 68.7 ± 4.45 |
| 50 µg/mL of *Centella asiatica* | 70.6 ± 0.96 |
| 100 µg/mL of *Centella asiatica* | 71.5 ± 1.87 |

As shown in Table 1, the 50% alcohol extract of *Centella asiatica* exhibited a cell survival rate of about 16% at 100 ug/mL as compared to the control, and protected cells from oxidative damage caused by A2E at the same level as that in lutein as a positive control. Therefore, the present invention may provide a composition for preventing, ameliorating, or treating glaucoma and macular degeneration for eye health including a *Centella asiatica* extract.

The foregoing description of the present invention is provided for illustrative purposes only, and it will be understood by those of ordinary skill in the art to which the present invention pertains that the present invention may be easily modified in other particular forms without changing the technical or essential characteristics of the present invention. Thus, the above-described embodiments should be construed as being provided for illustrative purposes only and not for purposes of limitation.

## Claims

1. A *Centella asiatica* extract for use in preventing, ameliorating, or treating a retinal nerve cell damage syndrome caused by oxidative stress, **characterized in that** the *Centella asiatica* extract is a 40%-60% aqueous ethanol extract.

2. The *Centella asiatica* extract for use according to claim 1, wherein the *Centella asiatica* extract improves the survival rate of retinal nerve cells or retinal pigment epithelial cells through increased sugar availability.

3. The *Centella asiatica* extract for use according to claim 1, wherein the *Centella asiatica* extract inhibits the apoptosis of retinal nerve cells.

4. The *Centella asiatica* extract for use according to claim 1, wherein the *Centella asiatica* extract prevents the death of retinal nerve cells from oxidative stress caused by A2E.

5. The *Centella asiatica* extract for use according to claim 1, wherein the *Centella asiatica* extract prevents or ameliorates a retinal nerve cell damage syndrome caused by oxidative stress.

6. The *Centella asiatica* extract for use according to claim 1, wherein the retinal nerve cell damage syndrome is macular degeneration or glaucoma.

7. The *Centella asiatica* extract for use according to any one of claims 1 to 6, wherein the *Centella asiatica* extract is administered at a dose of 0.01 mg/kg/day to 10 g/kg/day.

8. The *Centella asiatica* extract for use according to any one of claims 1 to 6, wherein the *Centella asiatica* extract is administered in a form of pharmaceutical composition, and the composition is a formulation for topical administration.

9. The *Centella asiatica* extract for use according to claim 8, wherein the composition is an ophthalmic formulation.

10. The *Centella asiatica* extract for use according to any one of claims 8 to 9, wherein the *Centella asiatica* extract is included in the composition at a concentration of 5 µg/ml to 500 µg/ml with respect to a total weight of the composition.

## Patentansprüche

1. Ein *Centella asiatica*-Extrakt zur Verwendung bei der Vorbeugung, Linderung oder Behandlung einer durch oxidativen Stress verursachten Schädigung von Nervenzellen der Netzhaut, **dadurch gekennzeichnet, dass** der *Centella asiatica*-Extrakt ein 40%-60%iger wässriger Ethanolextrakt ist.

2. *Centella asiatica*-Extrakt zur Verwendung nach Anspruch 1, wobei der *Centella asiatica-*Extrakt die Überlebensrate von Nervenzellen der Netzhaut oder Pigmentepithelzellen der Netzhaut durch erhöhte Zuckerverfügbarkeit verbessert.

3. *Centella asiatica*-Extrakt zur Verwendung nach Anspruch 1, wobei der *Centella asiatica-*Extrakt die Apoptose von Nervenzellen der Netzhaut hemmt.

4. *Centella asiatica*-Extrakt zur Verwendung nach Anspruch 1, wobei der *Centella asiatica-*Extrakt den Tod von Nervenzellen der Netzhaut durch oxidativen Stress, der durch A2E verursacht wird, verhindert.

5. *Centella asiatica*-Extrakt zur Verwendung nach Anspruch 1, wobei der *Centella asiatica-*Extrakt eine durch oxidativen Stress verursachte Schädigung von Nervenzellen der Netzhaut verhindert oder verbessert.

6. *Centella asiatica*-Extrakt zur Verwendung nach Anspruch 1, wobei die durch oxidativen Stress verursachte Schädigung von Nervenzellen der Netzhaut eine Makuladegeneration oder ein Glaukom ist.

7. *Centella asiatica*-Extrakt zur Verwendung nach einem der Ansprüche 1 bis 6, wobei der *Centella asiatica*-Extrakt in einer Dosis von 0,01 mg/kg/Tag bis 10 g/kg/Tag verabreicht wird.

8. *Centella asiatica*-Extrakt zur Verwendung nach einem der Ansprüche 1 bis 6, wobei der *Centella asiatica*-Extrakt in Form einer pharmazeutischen Zusammensetzung verabreicht wird und die Zusammensetzung eine Formulierung zur topischen Verabreichung ist.

9. *Centella asiatica*-Extrakt zur Verwendung nach Anspruch 8, wobei die Zusammensetzung eine ophthalmische Formulierung ist.

10. *Centella asiatica*-Extrakt zur Verwendung nach einem der Ansprüche 8 bis 9, wobei der *Centella asiatica*-Extrakt in der Zusammensetzung bezogen auf das Gesamtgewicht der Zusammensetzung in einer Konzentration von 5 µg/ml bis 500 µg/ml enthalten ist.

## Revendications

1. Extrait de *Centella asiatica* destiné à être utilisé pour prévenir, améliorer ou traiter un syndrome de lésions des cellules nerveuses rétiniennes causé par un stress oxydatif, **caractérisé en ce que** l'extrait de *Centella asiatica* est un extrait éthanolique aqueux à 40%-60%.

2. Extrait de *Centella asiatica* destiné à être utilisé selon la revendication 1, où l'extrait de *Centella asiatica* améliore le taux de survie des cellules nerveuses rétiniennes ou des cellules épithéliales pigmentaires rétiniennes en augmentant la disponibilité des sucres.

3. Extrait de *Centella asiatica* destiné à être utilisé selon la revendication 1, où l'extrait de *Centella asiatica* inhibe l'apoptose des cellules nerveuses rétiniennes.

4. Extrait de *Centella asiatica* destiné à être utilisé selon la revendication 1, où l'extrait de *Centella asiatica* prévient la mort des cellules nerveuses rétiniennes due au stress oxydatif causé par l'A2E.

5. Extrait de *Centella asiatica* destiné à être utilisé selon la revendication 1, où l'extrait de *Centella asiatica* prévient ou améliore un syndrome de lésions des cellules nerveuses rétiniennes causé par un stress oxydatif.

6. Extrait de *Centella asiatica* destiné à être utilisé selon la revendication 1, où le syndrome de lésions des cellules nerveuses rétiniennes est une dégénérescence maculaire ou un glaucome.

7. Extrait de *Centella asiatica* destiné à être utilisé selon l'une quelconque des revendications 1 à 6, où l'extrait de *Centella asiatica* est administré à une dose de 0,01 mg/kg/jour à 10 g/kg/jour.

8. Extrait de *Centella asiatica* destiné à être utilisé selon l'une quelconque des revendications 1 à 6, où l'extrait de *Centella asiatica* est administré sous forme de composition pharmaceutique, et la composition est une formulation destinée à l'administration topique.

9. Extrait de *Centella asiatica* destiné à être utilisé selon la revendication 8, où la composition est une formulation ophtalmique.

10. Extrait de *Centella asiatica* destiné à être utilisé selon l'une quelconque des revendications 8 à 9, où l'extrait de *Centella asiatica* est incorporé dans la composition à une concentration de 5 pg/ml à 500 µg/ml par rapport au poids total de la composition.
